# FASCICULE DE BREVET EUROPEEN

(11) **EP 0 371 347 B1**
(45) Date de publication et mention de la délivrance du brevet: **01.02.1995**
(21) Numéro de dépôt: 89121262.3
(22) Date de dépôt: 17.11.1989
(51) Int. Cl.: C07D 313/04, A23L 1/22

(54) **Ethers cycliques et leur utilisation à titre d'ingrédients parfumants ou aromatisants**
Zyklische Äther und deren Verwendung als Duft- oder Aromazutat
Cyclic ethers and their use as perfuming or flavouring ingredients

(30) Priorité: 28.11.1988 CH 4414/88
(43) Date de publication de la demande: 06.06.1990
(73) Titulaire: FIRMENICH SA, CH-1211 Genève 8 (CH)
(72) Inventeur: Morris, Anthony Francis, CH-1261 Gingins (CH); Naef, Regula, CH-1227 Carouge (CH); Escher, Sina, CH-1232 Confignon (CH); Velluz, Alain, F-74800 La Roche/Foron (FR)
(74) Mandataire: Salvadori, Giuseppe

(56) Documents cités:
- CHEMICAL ABSTRACTS, vol. 77, 1972, page 424, résumé no. 139803x, Columbus,Ohio, US; & JP-A-72 27 511 (T. SHONO) 22-07-1972

## Description

La présente invention a trait au domaine de la parfumerie et de l'industrie des arômes. Elle concerne en particulier des éthers cycliques de structure jusqu'ici inconnue, représentés par la formule que voici : 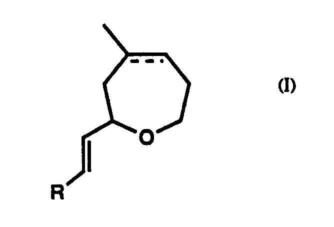
dans laquelle la ligne pointillée indique l'emplacement d'une liaison simple ou double et le symbole R représente un radical isopropényle ou un radical 1-éthoxy-1-méthyléthyle.

Nous avons découvert de façon surprenante que les éthers en question étaient dotés de propriétés odorantes très appréciées. En effet, ils servent à développer des notes florales, quelque peu fruitées, voire métalliques.

Les éthers de formule (I) sont des composés d'origine naturelle. Nous avons en effet découvert que ces composés étaient présents dans des quantités infimes, de l'ordre de 50 ppm (parties par million) dans les fruits du cognassier (Cydonia oblonga Mill.) et/ou dans de l'eau-de-vie obtenue par distillation du jus de coings fermenté.

Pour leur extraction, nous avons eu recours à un processus de codistillation dans un appareil de type dit de Likens-Nickerson à partir de fruits frais suivie par un fractionnement par chromatographie sur une colonne de gel de silice et une élution au moyen d'un mélange pentane/diéthyléther (9:1). L'identification a été ensuite effectuée à l'aide de différents moyens spectroscopiques, dont la spectrométrie de masse et la résonance magnétique nucléaire.

L'isolation à partir de l'eau-de-vie de coings s'est effectuée par contre au moyen d'une extraction dans un appareil dit de Kutscher-Steudel.

Les détails des méthodes suivies seront indiqués dans la partie descriptive qui suit.
a. Codistillation
   1,4 kg de fruits frais préalablement coupés en forme de petits cubes ont été broyés dans 2,250 l d'eau dans un appareil mixer et soumis à distillation dans un appareil de Likens-Nickerson [voir Proc. Am. Soc. Brew. Chem. 5, (1964)]. Il s'agit en fait d'un appareil modifié selon les indications décrites dans J. Agr. Food Chem. 25, 1946 (1977) par T. Schultz et al. La distillation a lieu en présence de 150 ml de pentane et eue se déroule pendant 5 heures. Les extraits ainsi obtenus ont été séchés sur du sulphate de magnésium, puis concentrés dans un appareil de distillation muni d'une colonne de type Vigreux. Le produit ainsi obtenu sous forme de résidu a été fractionné par chromatographie sur une colonne remplie de gel de silice en utilisant comme éluant un mélange de pentane et éther diéthylique (9:1). Le 2,3,6,7-tétrahydro-4-méthyl-2-(3-méthyl-1,3-butadiényl)oxépine, ou 4-méthyl-2-(3-méthyl-1,3-butadiényl)-1-oxa-4-cycloheptène, a pu ainsi être isolé dans la fraction apolaire. L'analyse gaz-chromatographique a montré que les temps de rétention étaient de 26'40'' sur une colonne CARBOWAX [Supelco®, 60 m/0,25 mm ; 80-250°C ;
   ΔT = 5°/min ; débit : 1,2 kg He] et
   18'50'' sur une colonne apolaire SPB [Supelco®, 60 m/0,25 mm ; 80-250°C ;
   ΔT = 5°/min ; débit : 1,5 kg He].
   Données analytiques du 2,3,6,7-tétrahydro-4-méthyl-2-(3-méthyl-1,3-butadiényl)oxépine ainsi isolé :
   - SM :: 178(M⁺,2) ; m/z : 163(4), 135(5), 110(4), 96(7), 82(35), 67(100), 53(8), 41(7)
   - ¹H-RMN (360MHz) :: 1,77(3H,s) ; 1,84(3H,s) ; 2,10(1H,d,J=16Hz) ; 2,18(1H,m) ; 2,40(1H,dxd, J₁=9Hz, J₂=16Hz) ; 2,55(1H,dxd,J₁=16Hz, J₂=11Hz) ; 3,55(1H,dxd, J₁=9Hz, J₂=12Hz) ; 4,05(2H,m) ; 4,98(2H,m) ; 5,60(1H,m) ; 5,70(1H,dxd,J₁=16Hz, J₂=7Hz) ; 6,33(1H,d,J=16Hz) δ ppm.
b. Extraction Kutscher-Steudel
   4,9 litres d'eau-de-vie de coings [produit commercial ; origine : "Coing du Feuillu", Saconnex-d'Arve, Genève, réserve (1982)] ont été dilués dans 4 l d'eau et extraits en continu avec 1 l d'éther diéthylique pendant 20 heures dans un appareil de type Kutscher-Steudel [voir : Hoppe-Seyler's Z. physiolog. Chem. 39, 473 (1903)].
   La solution éthérée a été ensuite concentrée dans un appareil à distiller muni d'une colonne Vigreux et la procédure a été répétée derechef sur une nouvelle quantité de 5 l d'eau-de-vie.
   Les résidus obtenus provenant des deux extractions (25,5 g) contenant surtout de l'éthanol ont été fractionnés par distillation sous vide à 7,98-5,32 Pa. A cette pression, on élimine l'alcool à 24-25°C et l'on obtient 1,05 g d'un extrait qui est soumis à séparation chromatographique sur colonne et fractionnement suivant la méthode indiquée sous lettre a. ci-dessus.
   Le 2,3,6,7-tétrahydro-4-méthyl-2-(3-méthyl-1,3-butadiényl)oxépine obtenu dans cette extraction s'est révélé en tous points identique au produit obtenu et décrit au paragraphe a. ci-dessus.
   D'autres composés ont également été séparés et leurs structures identifiées d'après leurs données analytiques comme présenté ci-après :
   4-méthyl-2-(3-méthyl-1,3-butadiényl)oxépane ou 4-méthyl-2-(3-méthyl-1,3-butadiényl)-1-oxa-cycloheptane
   Deux isomères ont été identifiés comme suit :

### isomère A :

Temps de rétention sur colonne CARBOWAX [conditions définies en a.] : 24'25''
- SM :: 180(M⁺,40) ; m/z : 165(82), 151(6), 137(17), 123(12), 111(43), 96(53), 81(52), 69(100), 55(96), 41(87).

### isomère B :

Temps de rétention sur colonne CARBOWAX [conditions définies en a.] : 25'00''
- SM :: 180(M⁺,41) ; m/z :165(90), 151(6), 137(17), 123(12), 111(45), 96(62), 81(73), 69(100), 55(72), 41(95).

### 2-(3-éthoxy-3-méthyl-1-butényl)-2,3,6,7-tétrahydro-4-méthyloxépine ou 2-(3-éthoxy-3-méthyl-1-butényl)-1-oxa-4-cycloheptène

Temps de rétention sur colonne CARBOWAX [conditions définies en a.] : 26'35''
- SM :: 224(M⁺,0,5) ; m/z : 209(4), 178(14), 163(6), 155(8), 142(5), 135(7), 113(13), 97(8), 87(18), 82(54), 67(100), 43(28).

Les structures déduites pour ces composés naturels à partir de leurs données analytiques citées ci-dessus sont définies à l'aide de la formule (I) précitée et ont été confirmées par synthèse, comme il est décrit plus loin. Il s'agit d'un type de structure peu courant et à ce jour inconnu pour des composés parfumants d'origine naturelle.

Selon l'invention, les composés de formule (I) peuvent être employés en tant qu'ingrédients actifs dans des compositions parfumantes, des parfums ou des produits parfumés. Ces derniers peuvent être de nature fort diverse ; il s'agit par exemple de savons, de détergents solides ou liquides, cationiques, anioniques ou zwitterioniques, d'adoucisseurs, de produits d'entretien, de désodorisants d'air ambiant ou des désodorisants corporels. On peut mentionner également à titre de produits parfumés, les cosmétiques ou les produits capillaires.

Les éthers cycliques (I) de l'invention trouvent également une utilisation particulière dans la reconstitution des huiles essentielles, notamment de type floral.

En dépit des très légères différences structurelles, les composés de formule (I) possèdent des propriétés olfactives qui varient de façon significative d'un composé à l'autre. C'est ainsi que l'odeur du 2,3,6,7-tétrahydro-4-méthyl-2-(3-méthyl-1,3-butadiényl)oxépine peut être définie comme florale, rosée. Par certains de ses caractères olfactifs, cet éther cyclique insaturé rappelle l'oxyde de rose, mais il se distingue de celui-ci par une note plus riche, plus complète et plus douce, qui est également une note puissante, d'un excellent pouvoir de diffusion.

Pour sa part, le 4-méthyl-2-(3-méthyl-1,3-butadiényl)oxépane (mélange de deux isomères) développe une note odorante de type floral, vert de rose, oxyde de rose, moins fruitée, moins puissante et caractéristique que celle de son analogue insaturé susmentionné. Lors de la préparation synthétique de ce composé, il a été possible d'évaluer séparément les deux isomères A et B cités précédemment et représentés par la formule 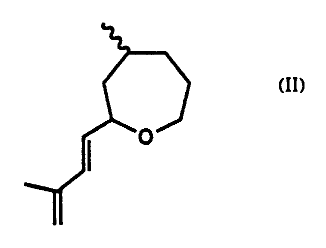
dans laquelle la ligne ondulée définit une liaison C-C de configuration cis ou trans. On a constaté que l'isomère A possédait une note odorante de type vert, floral, avec un agréable caractère amère rappelant la jacinthe, aussi grasse et fruitée, tandis que l'isomère B développait une odeur verte, métallique, plus puissante que l'odeur de l'isomère A.

Le 2-(3-éthoxy-3-méthyl-1-butényl)-2,3,6,7-tétrahydro-4-méthyloxépine possède, lui, une note odorante de type floral, vert, rose, jacinthe, avec une nuance vert amère de feuille.

Lorsqu'ils sont utilisés à titre d'ingrédients parfumants, les composés de formule (I) sont employés à des concentrations pouvant varier dans une gamme de valeurs très étendue. L'homme du métier sait par expérience que de telles valeurs sont déterminées en fonction de l'effet olfactif recherché, de la nature des autres coingrédients dans une composition donnée et naturellement de la nature du produit que l'on désire parfumer.

Du point de vue gustatif, les composés de l'invention présentent également des propriétés distinctes. C'est ainsi que le 2,3,6,7-tétrahydro-4-méthyl-2-(3-méthyl-1,3-butadiényl)oxépine possède une note aromatique boisée, verte, du type géranium, avec une nuance dans la direction de l'oxyde de rose, et fruitée, du type mangue, tandis que son analogue saturé ou 4-méthyl-2-(3-méthyl-1,3-butadiényl)oxépane se caractérise par une note aromatique rosée, grasse et verte, légèrement sale, métallique et légèrement fermentée, avec une nuance de fruit tropical.

Lorsqu'utilisés à titre d'agents aromatisants, ces composés (I) peuvent être employés dans des concentrations variées mais bien inférieures aux concentrations dans lesquelles ils sont utilisés pour les applications en parfumerie susmentionnées. Parmi les produits que l'on peut aromatiser à l'aide des produits de l'invention figurent bien entendu les aliments et les boissons. Toutefois, les composés de formule (I) peuvent également être utilisés pour conférer, améliorer ou modifier l'arôme et le goût de produits aussi variés que le tabac, les épices ou la gomme à mâcher.

Les composés de l'invention peuvent être préparés selon un procédé qui fait également l'object de la présente invention. Ce procédé est caractérisé en ce qu'on fait réagir un composé de formule 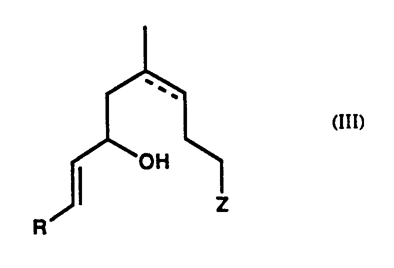
dans laquelle la ligne pointillée indique l'emplacement d'une liaison simple ou double, le symbole Z représente un groupe sortant dans les conditions de la réaction et le symbole R est défini comme à la revendication 1, avec une base forte, en présence d'un solvent organique inerte.

La réaction qui caractérise le procédé de l'invention est, en effet, une cyclisation ou éthérification intramoléculaire, effectuée par l'action d'une base forte et en présence d'un solvent organique inerte capable de stabiliser l'alcoolate formé dans ladite réaction. En tant que base forte dans ce procédé, on peut utiliser une base minérale ou organique telle qu'un hydrure ou un alkoxyde de métal alcalin, le sodium et le potassium de préférence. Parmi lesdites bases, il convient de citer l'hydrure de sodium ou potassium et le tert-butylate de sodium ou potassium.

Comme indiqué plus haut, la réaction s'effectue dans un solvent organique inerte. A cet effet, on peut utiliser des solvents organiques tels des éthers, comme le tétrahydrofuranne (THF) ou l'éther diméthylique de l'éthylèneglycol (monoglyme) en mélange avec des amides telles l'hexaméthyltriamidure de phosphore (HMPT) ou encore la N,N'-diméthyl-N,N'-propylèneurée [DMPU ; voir T. Mukhopadhyary et D. Seebach, Helv. Chim. Acta 65, 385 (1982)].

Selon un mode d'exécution préféré du procédé de l'invention, on utilise de l'hydrure de sodium en tant que base forte, la réaction s'effectuant dans un mélange de monoglyme et de DMPU.

A la formule (III) définie précédemment, le symbole Z représente un groupe sortant dans les conditions de la réaction décrite ci-dessus, par exemple, un radical p-toluènesulfonyloxy.

Les composés de l'invention pour lesquels le symbole R, à la formule (I), représente un radical 1-éthoxy-1-méthyléthyle peuvent également être préparés à partir des composés (I) pour lesquels le symbole R représente un radical isopropényle, par réaction de ces derniers avec l'éthanol, en présence d'acide p-toluènesulfonique.

Les composés de formule (III) définie précédemment, utilisés à titre de produits de départ dans le procédé selon l'invention, peuvent être obtenus selon un procédé à plusieurs étapes qui peut être représenté par le schéma que voici : 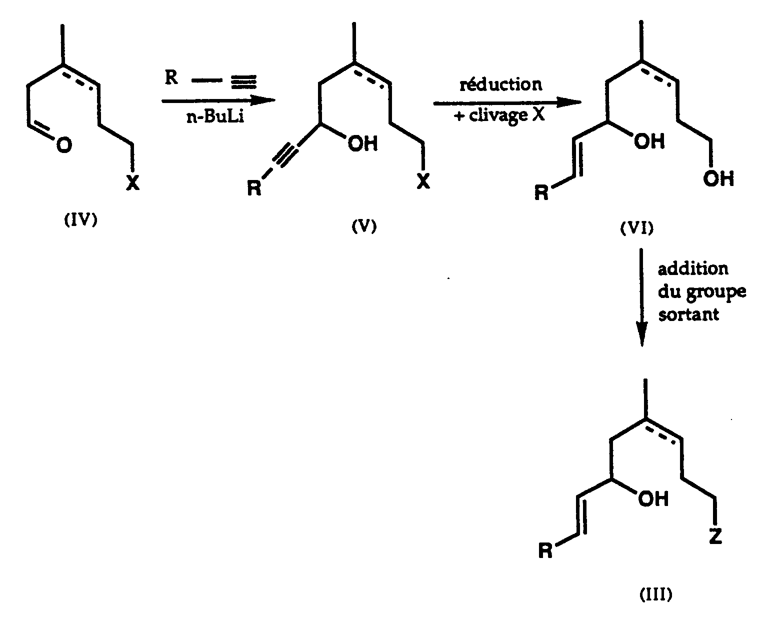
Dans ce schéma, la ligne pointillée indique partout l'emplacement d'une liaison simple ou double, le symbole R aux formules (III), (V) et (VI) représente un radical isopropényle ou un radical 1-éthoxy-1-méthyléthyle, le symbole X aux formules (IV) et (V) représente un groupe protecteur dans les conditions des réactions indiquées, par exemple, un radical acétyloxy ou tétrahydro-2(2H)-pyranyloxy, et le symbole Z à la formule (III) est défini comme auparavant.

Les aldéhydes (IV) sont des composés de structure nouvelle qui peuvent être obtenus par des réactions de type standard à partir de produits connus, comme il est décrit en détail dans les exemples de préparation présentés plus loin. La réaction de ces aldéhydes avec le sel de lithium de l'alcynyle indiqué, dans des conditions connues (voir, par exemple, L. Brandsma in "Preparative Acetylenic Chemistry", Elsevier, Amsterdam, 2ème éd. 1988, page 82) fournit l'énynol (V) en rendement quantitatif. La phase suivante du procédé illustré comprend la réduction de cet énynol, par exemple à l'aide de LiAlH₄, qui, selon la nature du groupe protecteur X, peut être accompagné du clivage concomitant de ce dernier ou, le cas échéant, est suivie d'une hydrolyse, pour fournir le diol (VI). Ce dernier est alors converti en le produit (III) désiré de façon classique, par exemple à l'aide de chlorure de p-toluènesulfonyle, dans la pyridine.

Les conditions spécifiques des réactions illustrées dans le schéma ci-dessus sont décrites plus en détail dans les exemples de préparation présentés ci-après, dans lesquels les températures sont indiquées en degrés centigrades et les abréviations ont le sens usuel dans l'art.

L'invention sera également illustrée de façon plus détaillée à l'aide des exemples d'applications présentés plus loin.

### Exemple 1

### Préparation de (E)-2,3,6,7-tétrahydro-4-méthyl-2-(3-méthyl-1,3-butadiényl)oxépine

a) (Z)-2-méthyl-5-(tétrahydro-2(2H)-pyranyloxy)-2-pentén-1-ol
   On a suivi une méthode analogue à celle décrite par M. Schlosser et al. dans Synthesis 1971, 380 et E.J. Corey et al. dans J. Amer. Chem. Soc. 92, 226 (1970). Dans un ballon à 4 cols equipé d'une agitation magnétique, d'un réfrigérant, d'une entrée d'argon, d'une ampoule d'introduction et d'un thermomètre, on a chargé 55,6 g (150 mmole) de bromure d'éthyltriphénylphosphonium et 300 ml de THF (tétrahydrofuranne, fraîchement distillé en présence de LiAlH₄). Le ballon a été refroidi dans un bain d'eau glacée. Une solution de n-butyllithium dans l'hexane (1,44 M, 104 ml, 150 mmole) a été introduite goutte à goutte et la solution rouge foncée formée a été maintenue sous agitation pendant 1 h à température ambiante et ensuite refroidie à -70°. On a ajouté au mélange de la réaction 23,7 g (150 mmole) de 3-(tétrahydro-2(2H)-pyranyloxy)propanol [obtenu, par exemple, selon le procédé décrit par W. Kitchling et al. dans J. Org. Chem. 54, 3893 (1989)] dans le THF (71 ml) pendant 20 min de façon à ce que la température interne reste inférieure à -60°. La couleur intense a disparu. Après avoir agité le mélange de la réaction pendant 20 min à -70°, on a introduit sur 20 min un nouvel équivalent de n-butyllithium dans l'hexane. Le mélange, devenu alors noir, a été chauffé à -5° et une solution de formaldéhyde dans le THF (environ 0,7M, 450 ml, environ 315 mmole ; préparée juste avant l'emploi à -78° comme décrit par M. Schlosser et al., dans Synthesis 1971, 380) a été siphonnée dans le ballon réactionnel par la voie d'un tube capillaire en acier inoxydable. On a observé la décoloration du mélange de la réaction et la formation d'un précipité blanc. Ce mélange a été laissé sous agitation pendant la nuit, à température ambiante. On a ajouté 74 ml d'eau et, 2 h plus tard, concentré la solution orange à basse pression pour réduire le volume à 200 ml, dilué avec 350 ml d'eau et extrait à l'éther. La phase organique a été lavée à neutralité, séchée sur MgSO₄ et concentrée dans un rotavapeur à pression réduite. Le produit brut ainsi obtenu a été distillé sur une colonne Vigreux de 12 cm. Les fractions qui ont distillé à 60-90°/6,65x10 Pa ont fourni 20,3 g de l'alcool désiré à environ 80% pur. Ce produit a été combiné avec 22,1 g d'un produit obtenu dans un essai parallèle et redistillé sur une colonne Vigreux de 12 cm. On a obtenu ainsi 35,01 g de (Z)-2-méthyl-5-(tétrahydro-2(2H)-pyranyloxy)-2-pentén-1-ol à 95% pur.
   P.éb. 85-89°/6,65x10 Pa ; rend 58,3%.
   Un échantillon analytique de l'alcool susmentionné, obtenu par chromatographie gazeuse préparative, présentait les données analytiques suivantes :
   - IR (liq.) :: 3400, 1205, 1140, 1125, 1080, 1040, 905, 880, 820 cm⁻¹ ;
   - RMN (¹H,360MHz) :: 1,84(s,3H) ; 2,38(m,2H) ; 3,38, 3,51, 3,78, 3,84(4m,4H) ; 4,03, 4,09(AB,J=8,3Hz,1H) ; 4,61(m,1H) ; 5,35 (t,J=9,0Hz, 1H) δ ppm ;
   - SM :: 200(0,M⁺) ; m/z : 85(100), 43(20), 67(18), 57(17), 101(9), 116(2), 170(1).
b) (Z)-1-bromo-2-méthyl-5-(tétrahydro-2(2H)-pyranyloxy)-2-pentène
   Une solution de l'alcool préparé selon a) (8,0 g, 40 mmole) dans l'hexane (400 ml), contenant 4 ml de pyridine, a été traitée à -7° avec une solution de PBr₃ (4 ml, 42,4 mmole) dans l'hexane (80 ml) ajoutée goutte à goutte. Après avoir terminé cette introduction (60 min), le mélange a été agité encore pendant 30 min à -5°, et ensuite versé sur un mélange eau-glace. On a extrait à l'éther et traité la phase organique comme décrit en a). On a obtenu 4,40 g de bromure brut qui a été utilisé sans purification pour l'étape suivante de la synthèse.
   - RMN (¹H,360MHz) :: 1,85(s,3H) ; 2,38(m,2H) ; 3,44, 3,55, 3,76, 3,85(4m,4H) ; 4,00(s,2H) ; 4,60(m,1H) ; 5,44(t,J=7,6Hz,1H) δ ppm ;
c) (Z)-2-[2-méthyl-5-(tétrahydro-2(2H)-pyranyloxy)-2-pentényl]-1,3-dithiane
   Une solution de 1,3-dithiane (33 mmole) dans du THF anhydre (33 ml, fraîchement distillé en présence de LiAlH₄) a été traitée avec une solution de n-butyllithium dans l'hexane (1,45 M, 23 ml, 33,3 mmole) sous argon à -20°. Le mélange a été maintenu sous agitation à -20° pendant 75 min et ensuite refroidi à -70°. On a ajouté goutte à goutte une solution du bromure préparé selon b) (8,70 g, 33 mmole) dans le THF (11 ml) de façon à maintenir la température au-dessous de -45°. On a interrrompu le flux d'argon, fermé le ballon de la réaction et rangé ce dernier dans le congélateur (-20°) pour la nuit. Ensuite, on a laissé chauffer la température du mélange de la réaction à température ambiante, extrait à l'éther, et la phase organique a été traitée comme décrit auparavant. Le produit brut de la réaction (12,80 g) a été filtré sur silica-gel (100 g), en utilisant comme éluant un mélange 8:2 hexane-acétate d'éthyle. On a obtenu 8,50 g d'un produit qui, selon l'analyse chromatographique, contenait en plus du composé désiré d'autres composants non identifiés. Afin de purifier ce produit, on l'a combiné avec 8,00 g d'un produit obtenu dans un essai parallèle, et soumis l'ensemble à une chromatograhie à pression moyenne [colonne LOBAR® (origine : Merck), mélange 85:15 hexane-acétate d'éthyle comme éluant]. 12,85 g de produit pur ont ainsi été obtenus (rend. 65,5%).
   - IR (liq.) :: 1200, 1180, 1140, 1120, 1080, 1040, 990, 970, 910, 880, 820 cm⁻¹ ;
   - RMN (¹H,360MHz) :: 1,78(s,3H) ; 2,50(d,J=7,5 Hz,2H) ; 2,65(m,4H) ; 3,41, 3,50, 3,73, 3,87(4m,4H) ; 4,21(t,J=7,5 Hz,1H) ; 4,60(m,1H) ; 5,37(t,J=7,2Hz,1H) δ ppm ;
   - SM :: 302(1,M⁺) ; m/z : 85(100), 119(53), 43(20), 67(15), 57(14).
d) (Z)-3-méthyl-6-(tétrahydro-2(2H)-pyranyloxy)-3-hexénal
   On a suivi une méthode analogue à celle décrite par R.L. Markezich et al. dans J. Amer. Chem. Soc. 95, 4414 (1973) et W.S. Johnson et al., ibid. 98, 1039 (1976). A un mélange du dithiane préparé selon c) (6,25 g, 20,7 mmole) et de CaCO₃ anhydre en poudre (8,28 g, 82,8 mmole) dans de l'acétonitrile aqueux (1:4, 106 ml), maintenu sous argon et agitation vigoureuse, on a ajouté de l'iodure de méthyle fraîchement distillé (12,4 g, 199 mmole) goutte à goutte. Le mélange réactionnel a été agité et maintenu sous argon pendant la nuit, à température ambiante. Par la suite, on a extrait à l'éther et traité la phase organique comme décrit en a). Le produit brut a été distillé dans un four à boules à 130-140°/6,65x10 Pa. On a obtenu 3,80 g (rend. 86,6%) de l'aldéhyde susmentionné qui a été utilisé tel quel dans la marche suivante :
   - IR (liq.) :: 1715, 1210, 1140, 1130, 1080, 1040, 990, 910, 880, 820 cm⁻¹ ;
   - RMN (¹H,360MHz) :: 1,78(s,3H) ; 3,12(m,2H) ; 3,40, 3,50, 3,75, 3,86(4m,4H) ; 4,58(m,1H) ; 5,52(t,J=7,2Hz,1H) ; 9,60(t,J=1,8Hz,1H) δ ppm ;
   - SM :: (0,M⁺) ; m/z : 85(100), 67(22), 55(17), 101(11), 41(10), 93(10), 110(4), 128(1), 183(1).
e) (E)-2,7-diméthyl-10-(tétrahydro-2(2H)-pyranyloxy)-3-décyne-1,7-dién-5-ol
   A une solution de 3-méthyl-3-butén-1-yne (1,58 g, 24 mmole, obtenu selon L. Brandsma, réf. citée, pages 88 et 203) dans l'éther diméthylique de l'éthylèneglycol (monoglyme) (60 ml, fraîchement distillé en présence de LiAlH₄), traversée par un courant d'argon, on a ajouté goutte à goutte, à -20°, une solution de n-butyllithium dans l'hexane (1,6 N, 13,8 ml, 22 mmole) suivie, après 45 min à -20°, de l'addition, à -30°/-20°, d'une solution de l'aldéhyde préparé selon d) (3,80 g, 17,9 mmole) dans l'éther diméthylique de l'éthylèneglycol (19 ml). On a laissé monter la température du mélange à 0° en 60 min et ensuite à la température ambiante. Après 30 min, on a hydrolysé le mélange de la réaction en ajoutant une solution aqueuse saturée de NH₄Cl. L'extraction à l'éther, suivie du traitement de la phase organique comme décrit précédemment, a fourni 4,91 g (rend. 98,6%) du diénol désiré qui a été employé tel quel dans l'étape suivante.
   - RMN (¹H,360MHz) :: 1,82(s,3H) ; 1,88(s,3H) ; 3,40, 3,50, 3,83(3m,4H) ; 4,60(m,2H) ; 5,21, 5,28(2s,2H) ; 5,40(t,J=7,6Hz,1H) δ ppm.
f) (3E,7Z)-2,7-diméthyl-10-(tétrahydro-2(2H)-pyranyloxy)-1,3,7-décattién-5-ol
   A une suspension de LiAlH₄ (1,33 g, 35 mmole) dans du THF anhydre (135 ml, fraîchement distillé en présence de LiAlH₄), on a ajouté goutte à goutte une solution du diénol préparé selon e) (4,91 g, 35 mmole) dans le THF (67 ml). On a porté à reflux pendant 60 min, après quoi l'analyse chromatographique sur couche mince a montré l'absence totale dudit diénol dans le mélange réactionnel. Ce dernier a été refroidi et hydrolysé avec soin à l'aide de glace et ensuite une solution aqueuse saturée de NH₄Cl. Par l'extraction à l'éther suivie du traitement usuel de la phase organique, on a obtenu 4,90 g de produit brut (rend. 99,2%), suffisamment pur pour la poursuite de la synthèse.
   - RMN (¹H,360MHz) :: 1,78(s,3H) ; 1,85(s,3H) ; 3,40, 3,50, 3,78, 3,84(4m,4H) ; 4,31(m,1H) ; 4,58(m,1H) ; 4,97(s,2H) ; 5,37(m,1H) ; 5,71(dxd,J₁=14,4Hz, J₂=7,2Hz, 1H) ; 6,36(d,J=14,4Hz,1H) δ ppm.
g) (3Z,7E)-4,9-diméthyl-3,7,9-décatriène-1,6-diol
   Une solution du composé préparé selon f) (4,90 g, 17,5 mmole) dans le méthanol (39 ml) a été traitée avec une solution aqueuse à 10% de HCl (10 ml) pendant 40 min. Après l'extraction à l'éther, la phase organique a été traitée comme décrit précédemment pour fournir 3,60 g de produit brut. Ce dernier a été purifié par chromatographie à pression moyenne (colonne LOBAR® et, comme éluant, hexane/acétate d'éthyle 1:1). On a obtenu 2,08 g (rend. 60,6%) du diol désiré à l'état semi-cristallin.
   - IR (liq.) :: 3300, 1610, 1120, 1060, 980, 890 cm⁻¹ ;
   - UV(MeOH) :: 228 nm (ε=16584) ;
   - RMN (¹H,360MHz) :: 1,80(s,3H) ; 1,85(s,3H) ; 3,59(dxdxd,J₁=J₂=10,4Hz, J₃-4,3Hz,1H) ; 3,71(dxdxd,J₁=10,4Hz, J₂=J₃=5,4Hz,1H) ; 4,35(m,1H) ; 4,98(s,2H) ; 5,35(t,J=8,6Hz,1H) ; 5,70(dxd,J₁=14,4Hz, J₂=5,8Hz,1H) ; 6,34(d,J=14,4Hz,1H) δ ppm ;
   - SM :: 196(0,M⁺) ; m/z : 97(100), 67(33), 69(23), 41(19), 79(16), 55(13), 105(5), 127(4), 119(3), 145(2), 178(1).
h) (E)-2,3,6,7-tétrahydro-4-méthyl-2-(3-méthyl-1,3-butadiényl)oxépine
   A une solution glacée du diol préparé selon g) (2,00 g, 10,2 mmole) dans la pyridine (22 ml), on a ajouté du chlorure de p-toluènesulfonyle (2,13 g, 11,2 mmole), en petites portions pendant 30 min. Le mélange a été agité pendant 30 min à 0°C et ensuite laissé au repos pendant la nuit à 3°. L'extraction à l'éther, suivie du traitement usuel de la phase organique déjà décrit, a fourni 2,85 g d'un produit brut qui, lors de l'analyse par chromatographie en couche mince, s'est avéré contenir deux composants. La purification supplémentaire de ce produit par chromatographie à pression moyenne (colonne LOBAR® et, comme éluant, hexane/acétate d'éthyle) a donné 2,20 g (rend. 61,6%) du produit voulu, ou p-toluènesulfonate de 6-hydroxy-4,9-diméthyl-3,7,9-décatriényle, et 220 mg d'un produit secondaire non identifié.
   On a préparé, sous argon, une suspension d'hydrure de sodium (à environ 80%, 630 mg, environ 21 mmole ; lavé au préalable avec du pentane anhydre) dans l'éther diméthylique de l'éthylèneglycol (30 ml, fraîchement distillé en présence de LiAlH₄) que l'on a refroidi à 0°. On a ajouté 900 »l (7,48 mmole) de DMPU (N,N'-diméthyl-N,N'-propylèneurée) et ensuite goutte à goutte une solution du p-toluènesulfonate susmentionné (2,20 g, 6,28 mmole) dans l'éther diméthylique de l'éthylèneglycol (30 ml). On a laissé monter la température du mélange réactionnel à température ambiante et laissé ce mélange sous agitation pendant la nuit. On a extrait à l'éther et traité la phase organique comme décrit sous a) pour obtenir 1,14 g de l'oxépine voulue à l'état brut. Ce produit a été purifié encore par chromatographie à pression moyenne (colonne LOBAR® et, comme éluant, un mélange hexane/éther 95:5) et ensuite par distillation au four à boules. On a obtenu 450 mg de (E)-2,3,6,7-tétrahydro-méthyl-2-(3-méthyl-1,3-butadiényl)oxépine.
   P. éb. 110-120°/14,63x10² Pa ; rend. 40,5%
   - IR (liq.) :: 1610, 1160, 1120, 1110, 1050, 970, 890 cm⁻¹ ;
   - UV(MeOH) :: 228 nm (ε=24865) ;
   - RMN (¹H,360MHz) :: 1,76(s,3H) ; 1,84(s,3H) ; 3,54(dxdxd,J₁=J₂=12,0Hz, J₃=1,0Hz,1H) ; 4,04(m,2H) ; 4,98(s,2H) ; 5,60(m,1H) ; 5,69(dxd,J₁=16,0Hz, J₂=6,5Hz,1H) ; 6,33(dxd,J=16,0HZ,1H) δ ppm ;
   - SM :: 178(2,M⁺) ; m/z : 67(100), 82(35), 81(12), 41(9), 53(9), 91(6), 135(4), 110(3), 163(3).

### Exemple 2

### Préparation de (E)-4-méthyl-2-(3-méthyl-1,3-butadiényl)oxépane

a) 6,6-diméthoxy-4-méthyl-1-hexanol
   Une solution de citronellal diméthyl acétal [55,0 g, 275 mmole, préparé à partir de citronellal racémique, selon le procédé décrit par V.R. Mamdapur et al. dans Tetrahedron 20, 2601 (1964)] dans le méthanol (550 ml) a été traitée à -75° avec un courant d'ozone pendant 3,5 h (4,5 g de O₃ par h, 328 mmole de O₃). L'excès d'ozone a été purgé à l'aide d'argon, on a laissé chauffer la solution à 0° et ensuite on l'a traitée avec une solution de NaBH₄ (5,22 g, 137,5 mmole) dans un mélange MeOH-H₂O (1:1, 154 ml). 2 h après, on a concentré le produit de la réaction à pression réduite. On a extrait à l'éther, lavé la phase organique à neutralité, séché sur MgSO₄ et concentré dans un rotavapeur à pression réduite. On a obtenu 60,2 g de l'hexanol désiré à environ 85% pur, qui a été utilisé tel quel dans la suite de la synthèse. Un échantillon analytique a été obtenu par chromatographie préparative en phase gazeuse :
   - IR (liq.) :: 3350, 1200, 1130, 1060 cm⁻¹ ;
   - RMN (¹H,360MHz) :: 0,93(d,J=6,1Hz,3H) ; 3,31(s,6H) ; 3,63(t,J=6,1 Hz,2H) ; 4,67(t,J=6,1Hz,1H) δ ppm ;
   - SM :: 176(0,M⁺) ; m/z : 75(100), 85(22), 61(18), 69(17), 41(12), 55(12), 95(8), 113(7), 145(3).
b) acétate de 6,6-diméthoxy-4-méthylhexyle
   L'alcool brut obtenu en a) (60,2 g) a été acétylé dans l'anhydride acétique (75 ml), en présence de pyridine (150 ml), pendant la nuit et à température ambiante. L'extraction à l'éther et le traitement de la phase organique [comme décrit en a)], suivis d'une distillation du produit brut sur colonne Vigreux, ont fourni 49,8 g de l'acétate désiré à 90% pur (rend. 74,8% ; p.éb. 120-125°/14,67x10² Pa).
   Un échantillon analytique a été obtenu par chromatographie préparative en phase gazeuse :
   - IR (liq.) :: 1740, 1250, 1140, 1060 cm⁻¹ ;
   - RMN (¹H,360MHz) :: 0,93(d,J=6,5Hz,3H) ; 2,05(s,3H) ; 3,32(s,6H) ; 4,05(t,J=7,2Hz, 2H) ; 4,46(t,J=6,1Hz,1H) δ ppm ;
   - SM :: 216(0,M⁺) ; m/z : 75(100), 85(39), 43(16), 95(13), 55(10), 113(4), 126(4), 187(1).
c) acétate de 5-formyl-4-méthylpentyle
   A une solution de l'acétate préparé selon b) (47,9 g, environ 200 mmole) dans l'acétone (880 ml), on a ajouté de l'eau (13,2 ml) et AMBERLYST® 15 (8,8 g ; origine : Rohm & Haas). Après avoir agité pendant 2 h, on a ajouté encore 6,6 ml d'eau et 4,4 g de résine, et 2 h plus tard, on a renouvelé cette addition. Lorsque la conversion de l'acétate était complète (environ 5,5 h), la suspension a été filtrée et concentrée à pression réduite. Le résidu a été dilué à l'éther, séché sur MgSO₄ et concentré. Le produit brut ainsi obtenu a été distillé sur une colonne Vigreux de 12 cm pour fournir 31,4 g de l'aldéhyde désiré à 90% pur (rend. : 83% ; p.éb. 115-120°/14,67x10² Pa).
   On a purifié par chromatographie en phase gazeuse un échantillon destiné à l'analyse :
   - IR (liq.) :: 1730, 1250, 1050 cm⁻¹ ;
   - RMN (¹H,360MHz) :: 0,98(d,J=6,5Hz,3H) ; 2.05(s,3H) ; 4,06(t,J=5,8Hz,2H) ; 9,77(t,J=1,0Hz,1H) δ ppm ;
   - SM :: 172(0,M⁺) ; m/z : 43(100), 69(88), 68(61), 61(57), 55(28), 56(25), 84(15), 97(15), 129(9).
d) (E)-4,9-diméthyl-7,9-décadiène-1,6-diol
   On a suivi le procédé décrit dans l'Exemple 1 e) en utilisant 14,4 g (218 mmole) de 3-méthyl-3-butén-1-yne dans 400 ml d'éther diméthylique de l'éthylèneglycol, 121 ml de solution de n-butyllithium dans l'hexane (1,6N, 197 mmole) et 30,96 g de l'aldéhyde préparé selon c) (environ 90% pur, 160 mmole) dans 150 ml d'éther diméthylique de l'éthylène glycol. Après l'extraction à l'éther et le traitement de la phase organique, on a obtenu 47,50 g d'un produit contenant 58% de (E)-10-acétoxy-2,7-diméthyl-3-décyn-1-én-5-ol et deux autres produits secondaires. Le mélange a été dissous, sans être purifié, dans du THF (600 ml) et a été ajouté à une suspension de LiAlH₄ (11,0 g, 289 mmole) dans le THF, le procédé décrit dans l'Exemple 1 f) ayant été suivi. On a obtenu 35,0 g de produit brut qui a été soumis à chromatographie sur colonne de silica-gel (250 g, éluant : hexane/acétate d'éthyle 1:1). On a obtenu 20,5 g d'un mélange 1:1, à 93% pur, de deux formes diastéréoisomères du diol désiré. Une purification supplémentaire par chromatographie gazeuse n'a pas permis de séparer les deux isomères (rend. 57%).
   - IR (liq.) :: 3350, 3080, 1620, 980, 900 cm⁻¹ ;
   - UV(MeOH) :: 228 nm (ε=21780) ;
   - RMN (¹H,360MHz) :: 0,93, 0,95(2d,J=6,1Hz,3H) ; 1,85(s,3H) ; 3,63(t,J=5,8Hz,2H) ; 4,27(dxdxd,J₁=J₂=J₃=6,5Hz,1H) ; 4,98(s,2H) ; 5,62, 5,66(2 fois dxd, J₁=15,8Hz, J₂=6,5Hz,1H) ; 6,3(d,J=15,8Hz,1H) δ ppm ;
   - SM :: 198(0,M⁺) ; m/z : 69(100), 55(77), 97(85), 41(50), 83(61), 111(21), 129(20), 165(4), 180(3).
e) (E)-4-méthyl-2-(3-méthyl-1,3-butadiényl)oxépane
   On a suivi le procédé décrit dans l'Exemple 1 h) en utilisant 10,0 g (environ 50 mmole) du diol préparé selon d), 200 ml de pyridine et 10,1 g (53 mmole) de chlorure de p-toluènesulfonyle. Après l'extraction à l'éther et le traitement de la phase organique, on a obtenu 13,3 g de produit brut qui a été purifié par chromatographie pour fournir 8,52 g de p-toluènesulfonate de 6-hydroxy-4,9-diméthyl-7,9-décadiényle, ainsi que 1,65 g d'un produit secondaire non identifié.
   On a continué comme décrit à l'Exemple 1 h) en utilisant 2,68 g de NaH (111 mmole) dans 130 ml d'éther diméthylique de l'éthylèneglycol, 3,80 ml de DMPU (31,6 mmole) et 8,52 g (24 mmole) du p-toluènesulfonate susmentionné dans 130 ml d'éther diméthylique de l'éthylèneglycol. Après purification de 5,1 g de produit brut, on a obtenu 3,0 g d'un mélange à plus de 98% pur, contenant (E)-4-méthyl-2-(3-méthyl-1,3-butadiényl)oxépane sous deux formes diastéréoisomères (environ 1:1).
   P.éb. 111-112°/14,67x10² Pa ; rend. 69%.
   Les deux diastéréoisomères ont été séparés par chromatographie gazeuse préparative, effectuée à plusieurs reprises.

### Diastéréoisomère A

- IR (liq.) :: 3090, 1615, 980, 900 cm⁻¹ ;
- UV(MeOH) :: 228 nm (ε=25527) ;
- RMN (¹H,360MHz) :: 0,98(d,J=6,8Hz,3H) ; 1,84(s,3H); 1,95(m,1H) ; 3,53(m,1H) ; 3,85(m,1H) ; 4,20(dxdxd,J₁=J₂=J₃=6,1Hz,1H) ; 4,95(s,2H) ; 5,68(dxd, J₁=15,1Hz, J₂=6,1Hz,1H) ; 6,29(d,J=15,1Hz,1H) δ ppm ;
- RMN (¹³C, 90,5MHz) :: 18,6(q) ; 23,2(q) ; 29,9(d) ; 30,6(t) ; 36,1(t) ; 43,1(t) ; 69,5(t) ; 77,7(d) ; 116,1(t) ; 131,9(d) ; 132,0(d) ; 141,7(s) δ ppm ;
- SM :: 180(30,M⁺) ; m/z : 69(100), 41(100), 165(78), 55(75), 81(61), 96(57), 111(44), 137(17), 121(21), 151(5).

### Diastéréoisomère B

- IR (liq.) :: 3090, 1615, 980, 900 cm⁻¹ ;
- UV(MeOH) :: 228 nm (ε=27964) ;
- RMN (¹H,360MHz) :: 0,97(d,J=6,5Hz,3H) ; 1,84(s,3H) ; 3,78(m,2H) ; 4,08(dxdxd,J₁=10,8Hz, J₂=6,1Hz, J₃=2,1Hz,1H) ;4,95(s,2H) ; 5,67(dxd, J₁=16,2Hz, J₂=6,1Hz,1H) ; 6,28(d,J=16,2Hz,1H) δ ppm ;
- RMN (¹³C, 90,5MHz) :: 18,5(q) ; 23,9(q) ; 29,0(t) ; 33,6(d) ; 35,0(t) ; 45,4(t); 67,3(t) ; 78,2(d) ; 116,1(t) ; 131,7(d) ; 131,9(d) ; 141,7(q) δ ppm ;
- SM :: 180(33,M⁺) ; m/z : 69(100), 41(100), 55(82), 165(80), 81(63), 96(60), 111(43), 137(15), 123(12), 151(5).

### Exemple 3

### Préparation de 2-(3-éthoxy-3-méthyl-1-butényl)-2,3,6,7-tétrahydro-4-méthyl-oxépine

On a chauffé à 50°C, pendant 3,5h, 50 mg de l'oxépine préparée selon l'Exemple 1 et 20 mg d'acide p-toluènesulfonique dans 10 ml d'éthanol. Le mélange de la réaction a été dilué à l'eau et extrait à l'éther. On a obtenu un produit contenant 10% de l'oxépine désirée, qui a été séparée par chromatographie gazeuse préparative.
- RMN (¹H,360MHz) :: 1,16(t,J=7Hz,3H) ; 1,28(s,6H) ; 1,76(s,3H) ; 2,07(d,J=18Hz,1H) ; 2,15(m,1H) ; 2,39(t large,1H) ; 2,54(dxd,J₁=18Hz, J₂=11Hz,1H) ; 3,35(q, J=7Hz,2H) ; 3,53(dxd,J₁=J₂=11Hz,1H) ; 4,01(m,2H) ; 5,60(m,1H) ; 5,62(dxd, J₁=7Hz, J₂=16Hz,1H) ; 5,68(d,J=16Hz,1H) δ ppm ;
- SM :: 224(M⁺, 0,5) ; m/z : 209(4), 178(8), 163(4), 155(5), 142(3), 135(5), 113(13), 97(5), 87(11), 82(40), 67(100), 41(27).

### Exemple 4

### Composition parfumante de type fleuri-fruité

On a préparé une composition parfumante de base de type fleuri-fruité par mélange avec les ingrédients suivants :

| Ingrédient | Parties en poids |
|---|---|
| Citronellol | 2000 |
| Alcool phényléthylique | 2000 |
| IRALIA® ¹⁾ | 1000 |
| Phénylhexanol | 500 |
| Terpinéol | 500 |
| Linalol | 500 |
| 2-Méthylbutanoate de 2-phényléthyle | 500 |
| Décanoate d'éthyle | 500 |
| Essence de muguet synt. | 2000 |
| Total | 9̅5̅0̅0̅ |

| | |
|---|---|
| 1) méthylionone ; origine : Firmenich SA, Genève, Suisse | |

Lorsqu'on a ajouté à cette composition de base 0,5% en poids de (E)-2,3,6,7-tétrahydro-4-méthyl-2-(3-méthyl-1,3-butadiényl)oxépine ou de (E)-4-méthyl-2-(3-méthyl-1,3-butadiényl)oxépane, on a obtenu une nouvelle composition dont le caractère rose et fruité était beaucoup plus volumineux et puissant que celui de la composition de base. La note odorante développée par cette nouvelle composition était beaucoup plus riche et possédait un pouvoir de diffusion prononcé.

### Exemple 5

### Préparation d'un savon parfumé

A une masse de savon en copeaux obtenue à partir d'une base de savon au sodium préparée à partir d'huile de coco et de suif, on a ajouté 0,2% de l'un des composés selon l'invention cités dans l'exemple précédent. La note grasse savonneuse s'est trouvée alors supprimée et le savon ainsi parfumé dégageait une note rose-fruitée d'une puissance et pouvoir de diffusion rares.

### Exemple 6

### Composition aromatisante fruitée, type mangue

On a préparé une composition de base de type mangue en mélangeant les ingrédients suivants :

| Ingrédient | Parties en poids |
|---|---|
| Huile essentielle de Buchu à 10% * | 15 |
| Butylate d'éthyle | 100 |
| Caproate d'éthyle | 50 |
| γ-Décalactone | 30 |
| Butyrate de géranyle | 20 |
| Hexanal | 5 |
| cis-3-Hexénol | 10 |
| Acétate d'hexyle | 40 |
| Caproate d'hexyle | 40 |
| α-Ionone à 0,1% * | 10 |
| Cinnamate d'isobutyle | 30 |
| Orange TETRAROME® ¹⁾ | 20 |
| Citron TETRAROME® ²⁾ | 25 |
| Ethanol à 95% | 605 |
| Total | 1000 |

| | |
|---|---|
| * dans l'éthanol | |
| 1) huile essentielle d'orange déterpénée ; origine : Firmenich SA, Genève, Suisse | |
| 2) huile essentielle de citron déterpénée ; origine : Firmenich SA, Genève, Suisse | |

La base de type mangue ainsi obtenue a servi à préparer trois compositions aromatisantes par mélange avec les ingrédients suivants :

| Ingrédient | Compositions (parties en poids) | | |
|---|---|---|---|
| | A | B | C |
| Base mangue | 100 | 100 | 100 |
| (E)-2,3,6,7-tétrahydro-4-méthyl-2-(3-méthyl-1,3-butadiényl)oxépine | - | 3 | - |
| (E)-4-méthyl-2-(3-méthyl-1,3-butadiényl) oxépane | - | - | 2 |
| Ethanol à 95% | 900 | 897 | 898 |
| Total | 1000 | 1000 | 1000 |

Les compositions A, B et C ont été évaluées par un panel d'experts dans une solution sucrée acide (10% de sucre, 0,1% d'acide citrique) à 0,1% dans de l'eau de source.
De l'avis des experts, la composition B était plus fruitée et juteuse et possédait plus de volume que la composition A. De plus, elle possédait un caractère mangue plus naturel.
La composition C possédait le caractère boisé, fleuri et rosé typique de la mangue brésilienne qui ne se trouvait pas dans la composition A.
Ainsi, l'addition d'un composé selon l'invention à une composition de base type mangue a donné comme résultat une composition aromatisée améliorée, avec un caractère mangue plus naturel. De plus, chacun des composés de l'invention conférait un caractère mangue de qualité distincte à la composition de base.

## Revendications

1. Composé de formule 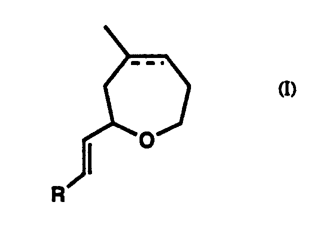 dans laquelle la ligne pointillée indique l'emplacement d'une liaison simple ou double et le symbole R représente un radical isopropényle ou un radical 1-éthoxy-1-méthyléthyle.

2. Composé de formule (I) selon la revendication 1 dans sa forme pratiquement pure et non accompagné des substances d'origine naturelle présentes dans le fruit du cognassier, ses extraits ou des fractions qui en dérivent.

3. A titre d'un composé selon la revendication 1, l'un des composés suivants :
a. (E)-2,3,6,7-tétrahydro-4-méthyl-2-(3-méthyl-1,3-butadiényl)oxépine
b. (E)-4-méthyl-2-(3-méthyl-1,3-butadiényl)oxépane.

4. A titre d'un composé de formule (I) selon la revendication 1, le (E)-4-méthyl-2-(3-méthyl-1,3-butadiényl)oxépane dans une de ses formes diastéréoisomères représentées par la formule 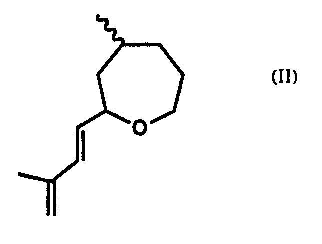 dans laquelle la ligne ondulée définit une liaison C-C de configuration cis ou trans.

5. Utilisation d'un composé selon la revendication 1 à titre d'ingrédient parfumant ou aromatisant.

6. Composition parfumante contenant à titre d'ingrédient odoriférant actif un composé selon la revendication 1.

7. Savon, détergent ou cosmétique contenant à titre d'ingrédient odoriférant actif un composé selon la revendication 1.

8. Composition aromatisante contenant à titre d'ingrédient aromatisant actif un composé selon la revendication 1.

9. Aliment ou boisson aromatisé contenant à titre d'ingrédient aromatisant actif un composé selon la revendication 1.

10. Procédé de préparation d'un composé de formule (I) selon la revendication 1, caractérisé en ce qu'on fait réagir un composé de formule 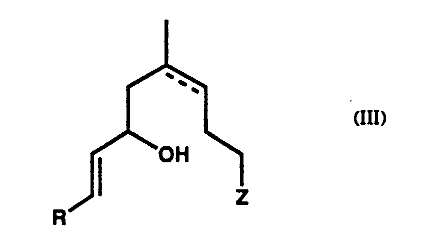 dans laquelle la ligne pointillée indique l'emplacement d'une liaison simple ou double, le symbole Z représente un groupe sortant dans les conditions de la réaction et le symbole R est défini comme à la revendication 1, avec une base forte, en présence d'un solvent organique inerte.

11. Procédé selon la revendication 10, caractérisé en ce que ladite base forte est l'hydrure de sodium et ledit solvent organique est un mélange d'éther diméthylique de l'éthylèneglycol et de N,N'-diméthyl-N,N'-propylèneurée.

12. Procédé selon la revendication 10, caractérisé en ce qu'on fait réagir un composé de formule (III) dans laquelle le symbole Z représente un radical p-toluènesulfonyloxy.

13. Utilisation d'un composé de formule (I) dans laquelle la ligne pointillée indique l'emplacement d'une liaison simple ou double et le symbole R représente un radical isopropényle, à titre de produit de départ pour la préparation d'un composé de formule (I) dans laquelle la ligne pointillée a le sens indiqué ci-dessus et le symbole R représente un radical 1-éthoxy-1-méthyléthyle, caractérisé en ce qu'on fait réagir ledit produit de départ avec l'éthanol, en présence de l'acide p-toluènesulfonique.

## Claims

1. Compound of formula 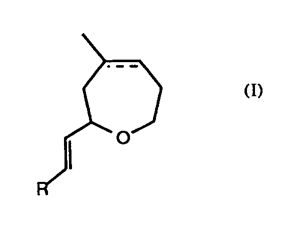 wherein the dotted line indicates the location of a single or double bond and the symbol R stands for an isopropenyl radical or a 1-ethoxy-1-methylethyl radical.

2. Compound of formula (I) according to claim 1, in its essentially pure form and free from the natural origin substances which are present in the quince fruit, its extracts or fractions derived therefrom.

3. As a compound according to claim 1, one of the following compounds :
a. (E)-2,3,6,7-tetrahydro-4-methyl-2-(3-methyl-1,3-butadienyl)oxepin ;
b. (E)-4-methyl-2-(3-methyl-1,3-butadienyl)oxepane.

4. As a compound of formula (I) according to claim 1, (E)-4-methyl-2-(3-methyl-1,3-butadienyl)oxepane in the form of one of its diastereomers represented by the formula 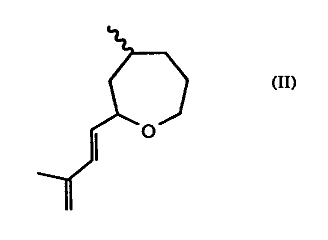 wherein the wavy line defines a C-C bond of cis or trans configuration.

5. Use of a compound according to claim 1 as a perfuming or flavouring ingredient.

6. Perfuming composition containing as an active odoriferous ingredient a compound according to claim 1.

7. A soap, a detergent or a cosmetic preparation containing as an active odorifierous ingredient a compound according to claim 1.

8. Flavouring composition containing as an active flavouring ingredient a compound according to claim 1.

9. A flavoured foodstuff or drink, containing as an active flavouring ingredient a compound according to claim 1.

10. Process for the preparation of a compound of formula (I) according to claim 1, characterized in that a compound of formula 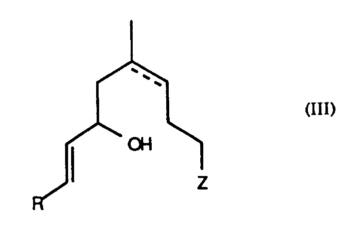 wherein the dotted line indicates the location of a single or double bond, the symbol Z represents a leaving group under the reaction conditions and the symbol R is defined as in claim 1, is reacted with a strong base, in the presence of an inert organic solvent.

11. Process according to claim 10, characterized in that said strong base is sodium hydride and said organic solvent is a mixture of ethyleneglycol dimethylether and N,N'-dimethyl-N,N'-propylene urea.

12. Process according to claim 10, characterized in that a compound of formula (III) wherein the symbol Z stands for a p-toluenesulfonyloxy radical is used in the reaction.

13. Use of a compound of formula (I) wherein the dotted line indicates the location of a single or double bond and the symbol R stands for an isopropenyl radical, as a starting product for the preparation of a compound of formula (I) wherein the dotted line is defined as above and the symbol R stands for a 1-ethoxy-1-methylethyl radical, characterized in that said starting product is reacted with ethanol, in the presence of p-toluenesulfonic acid.

## Patentansprüche

1. Verbindung der Formel 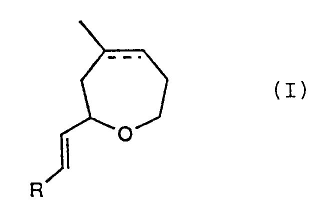 worin die punktierte Linie die Stelle einer Einfachbindung oder einer Doppelbindung anzeigt und das Symbol R für einen Isopropenyl-Rest oder einen 1-Ethoxy-methylethyl-Rest steht.

2. Verbindung der Formel (I) gemäss Patentanspruch 1 in ihrer praktisch reinen Form, die nicht begleitet ist von Substanzen natürlichen Ursprungs, die sich in der Frucht des Quittenbaumes, seinen Extrakten oder davon herstammenden Fraktionen befinden.

3. Als eine Verbindung gemäss Patentanspruch 1, eine der folgenden Verbindungen:
a. (E)-2,3,6,7-Tetrahydro-4-methyl-2-(3-methyl-1,3-butadienyl)oxepin
b. (E)-4-Methyl-2-(3-methyl-1,3-butadienyl)oxepan.

4. Als eine Verbindung der Formel (I) gemäss Patentanspruch 1 das (E)-4-Methyl-2-(3-methyl-1,3-butadienyl)oxepan in einer seiner diastereoisomeren Formen dargestellt durch die Formel 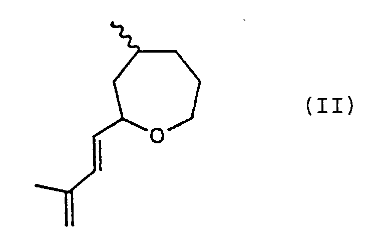 worin die Wellenlinie eine C-C Bindung der Cis- oder Trans-Konfiguration definiert.

5. Verwendung einer Verbindung gemäss Patentanspruch 1 als Riechstoffbestandteil oder Aromabestandteil.

6. Riechstoffkomposition, enthaltend als aktiven wohlriechenden Bestandteil eine Verbindung gemäss Patentanspruch 1.

7. Seife, Detergent oder Kosmetikum enthaltend als aktiven wohlriechenden Bestandteil eine Verbindung gemäss Patentanspruch 1.

8. Aromakomposition, enthaltend als aktiven aromatisierenden Bestandteil eine Verbindung gemäss Patentanspruch 1.

9. Aromatisiertes Lebensmittel oder Getränk enthaltend als aktiven aromatisierenden Bestandteil eine Verbindung gemäss Patentanspruch 1.

10. Verfahren zur Herstellung einer Verbindung der Formel (I) gemäss Patentanspruch 1, dadurch gekennzeichnet, dass man eine Verbindung der Formel 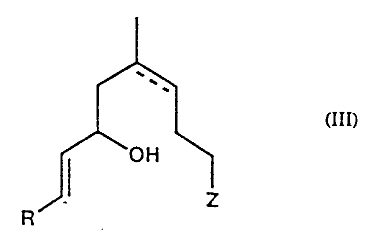 worin die punktierte Linie die Stelle einer Einfachbindung oder einer Doppelbindung anzeigt, das Symbol Z eine unter den Reaktionsbedingungen abspaltbare Gruppe bedeutet und das Symbol R wie im Patentanspruch 1 definiert ist, mit einer starken Base in Anwesenheit eines inerten organischen Lösungsmittels umsetzt.

11. Verfahren gemäss Patentanspruch 10, dadurch gekennzeichnet, dass die besagte starke Base Natriumhydrid ist und das besagte organische Lösungsmittel ein Gemisch des Ethylenglykoldimethylethers und des N,N'-Dimethyl-N,N'-propylenharnstoffes ist.

12. Verfahren gemäss Patentanspruch 10, dadurch gekennzeichnet, dass man die Verbindung (III), worin das Symbol Z einen p-Toluolsulfonyloxyrest bedeutet, einsetzt.

13. Verwendung einer Verbindung der Formel (I), worin die punktierte Linie die Stelle einer Einfachbindung oder Doppelbindung anzeigt und das Symbol R für einen Isopropenyl-Rest steht als Ausgangsverbindung für die Herstellung einer Verbindung der Formel (I), worin die punktierte Linie die oben angegebene Bedeutung besitzt und das Symbol R für einen 1-Ethoxy-1-methylethyl-Rest steht, dadurch gekennzeichnet, dass man diese Ausgangsverbindung mit Ethanol in Anwesenheit von p-Toluolsulfonsäure umsetzt.
